Europäisches Patentamt

European Patent Office

Office européen des brevets

(19)

(11) Publication number: **0 167 354**
**A1**

(12) # EUROPEAN PATENT APPLICATION

(21) Application number: 85304586.2

(22) Date of filing: 27.06.85

(51) Int. Cl.⁴: **B 01 J 23/20**

(30) Priority: 29.06.84 US 626066
29.06.84 US 626067

(43) Date of publication of application:
08.01.86 Bulletin 86/2

(84) Designated Contracting States:
BE DE FR GB IT NL

(71) Applicant: **Exxon Research and Engineering Company**
**P.O.Box 390 180 Park Avenue**
**Florham Park New Jersey 07932(US)**

(72) Inventor: **Chersich, Claudio Cimatti**
**467 Palisade Avenue**
**Englewood Cliffs New Jersey 07632(US)**

(72) Inventor: **Wachs, Israel Ephraim**
**340 Rolling Knolls Way**
**Bridgewater New Jersey 08807(US)**

(74) Representative: **Field, Roger Norton et al,**
**ESSO Engineering (Europe) Ltd. Patents & Licences Apex**
**Tower High Street**
**New Malden Surrey KT3 4DJ(GB)**

(54) Catalysts comprising niobia and/or tantala supported on titania.

(57) Catalysts comprising an oxide of a metal selected from (a) niobium, (b) mixtures of niobium and tantalum, and (c) tantalum, the oxide being supported on a titania support wherein at least a portion, and preferably at least about 25 wt.%, of said supported metal oxide is in a non-crystalline form. These catalysts have been found to be useful for synthesizing methanethiol and $CH_4$ from mixtures of $H_2S$ and CO. Niobia on tantala is a useful catalyst for removing $NO_x$ from industrial waste gases.

This invention relates to a catalyst composition of matter comprising an oxide of a metal selected from (a) niobium, (b) mixtures of niobium and tantalum or, (c) tantalum supported on a titania support wherein at least a portion of the supported metal oxide is in a non-crystalline form.

U.S. 4,149,998 to Tauster et al. relates to heterogeneous catalysts consisting of Group VIII metals, dispersed on oxide carriers selected from Ti, V, Nb, Ta and mixtures thereof and zirconium titanate and $BaTiO_3$. However, there is no suggestion in this patent that the niobium, vanadium or tantalum be supported on titania.

The usefulness of niobia supported on titania for the reduction of NO with $NH_3$ has been shown by Susumu Okazaki and Tetsuhiro Okuyama in "$Nb_2O_5$ Supported on $TiO_2$ Catalytic Activity for Reduction of NO with $NH_3$" which was published in Bull. Chem. Soc. Jpn., 56, 2159-2160 (July, 1983). In this work, Okazaki et al reacted NO with $NH_3$ in the presence of a niobia on titania catalyst over a temperature regime of from 200-500°C using a conventional flow reactor, with the mixture of NO and $NH_3$ being diluted with air. The catalytic activity of niobia supported on titania for the reduction of NO with $NH_3$ in the

presence of air was much higher than the catalytic activity of niobia supported on other supports such as alumina, zirconia, and silica.

The present invention concerns a catalyst compositions comprising an oxide of a metal selected from (a) niobium, (b) mixtures of niobium and tantalum or, (c) tantalum, said metal oxide being supported on titania wherein at least a portion of said supported metal oxide is in a non-crystalline form. These catalyst compositions are useful for synthesizing methanethiol, $CH_3SH$ and $CH_4$ from mixtures of $H_2S$ and CO. Moreover, niobia on titania is known to be a useful catalyst for reducing NO with $NH_3$ in the presence of air. Thus, niobia on tantala is a useful catalyst for removing NO from waste gases. The compositions of this invention are also useful as catalyst supports for supporting iron carbide or ruthenium for Fischer-Tropsch hydrocarbon synthesis reactions. In a preferred embodiment at least about 25 wt.% of the supported metal oxide will be in a non-crystalline form.

As previously stated, the catalyst compositions of the present invention comprise an oxide of a metal selected from (a) niobium, (b) mixtures of niobium and tantalum, or (c) tantalum, the metal oxide being supported on a titania support wherein at least a portion of the supported metal oxide, and preferably at least about 25 wt.% thereof, is in a non-crystalline form.

In the catalyst composition of this invention, the surface of the titania is modified by the oxide of niobium, mixture of niobium and tantalum or tantalum in an amount such that the modified surface of the so-modified titania exhibits properties different from titania whose surface has not been modified and also different from bulk oxides of niobium or tantalum and mixtures of bulk oxides of niobium and tantalum. Consequently, the catalyst compositions exhibit properties different from both unmodified titania and bulk oxides of niobium or tantalum or mixtures of bulk oxides of niobium and tantalum.

Thus, the catalysts of this invention comprise titania whose surface has been modified with an oxide of niobium or tantalum or a mixture of niobium and tantalum. That is, the surface of the titania has been modified by the supported oxides in an amount such that the catalyst exhibits properties different from titania whose surface has not been modified and different from bulk oxides of niobium or tantalum and mixture of oxides of niobium and tantalum. Those skilled in the art know that the oxides of niobium, tantalum and mixtures thereof are crystalline in their bulk form. Thus, at least a portion of and preferably at least about 25 wt.% of the supported metal oxide will be in a non-crystalline form. This will be accomplished if the metal oxide loading on the titania broadly ranges between about 0.5 to 25 wt.% of the total catalyst weight.

In preparing the catalyst compositions of this invention an oxide or precursor thereof of a metal selected from                          (a) niobium,  (b) mixtures of niobium and tantalum, or (c) tantalum is deposited on the titania to form either the surface modified titania catalyst composition of this invention or, in the case of one or more precursors, a catalyst precursor composite. The precursor composite is then calcined to oxidize the precursor composite to form a catalyst composition of this invention. The catalyst precursor composites of this invention may be prepared by techniques well-known in the art, such as incipient wetness, impregnation, etc., the choice being left to the practitioner. When using the impregnation technique, the impregnating solution is contacted with the support material for a time sufficient to deposit the precursor material onto the support either by selective adsorption or alternatively, the excess solvent may be evaporated during drying leaving behind the precursor salt. Advantageously, incipient wetness techniques may also be used. The choice of catalyst preparation is left to the practitioner. The transition metal oxide precursor salt solution used in preparing the catalyst of this invention may be aqueous or organic, the only requirement being that an adequate amount of precursor compound for the selected transition metal oxide be soluble in the solvent used in preparing this solution.

The final catalyst composite will then normally be dried at temperatures ranging from about 50°-300°C to remove the excess solvent and, if necessary decompose the salt if it is an organic salt. The transition metal oxide precursor is then converted into the oxide form by calcining at temperatures of from about 150° to 800°C and preferably 300°-700°C in a suitable oxidizing atmosphere such as air, oxygen, etc. The time required to calcine the composite will, of course, depend on the temperature and in general will range from about 0.5 to 7 hours. Reducing atmospheres may also be used to decompose the transition metal oxide precursors, but the resulting composite will then require subsequent calcination to convert the reduced metal component to the oxide form.

The catalysts of this invention will generally have metal oxide loadings of from about 0.5 to 25 wt.% metal oxide based on the total catalyst composition, preferably from about 1 to 15 wt.%, more preferably from about 2-10 wt.% based on the total catalyst composition.

The invention will be more readily understood by reference to the following Example.

Experimental

Hydrogen sulfide was obtained in compressed cylinders from Scientific Gas Products (electronic grade 99.999% purity), while carbon monoxide was purchased from Matheson Gas (99.99% purity). The above gases were checked for absence of hydrocarbon impurities (MS analysis and G.C. analysis with FID detector) and used without further purification. Helium (99.99%), when used as an inert carrier gas, was predried in a molecular sieve trap, scrubbed for $O_2$ removal in a hot Cu trap, and redried in a molecular sieve trap. Gas flows were regulated with Tylan F-260 flow controllers and premixed in a gas manifold system prior to entering into the catalyst bed.

A quartz reactor tube of 9 mm ID by 700 mm was loaded with 2.5 gram samples of -40/+60 mesh (Tyler) catalyst particles which were supported on each end with degreased quartz wool plugs. An external thermocouple was attached to the outside of the quartz tube near the center of the catalyst bed to record reaction temperature. The tube was heated with a three zone electric furnace (ATS-3210) equipped with Omega set point controllers.

Product analysis was accomplished with an on-line Carle GC (series SX) equipped with a hydrogen transfer system and FID/TC detectors. Gas phase samples were also separated on a Perkin Elmer 900 GC coupled to a DuPont 21-491 mass spectrometer for product identification of $CH_3SH$ and $CH_3SCH_3$. Response factors for quantitative G.C. analysis were obtained from a primary standard mixture of gases with quantities similar to the product mixture.

Catalyst Preparation

Degussa P-25, a mixture of anatase and rutile titania, was used as the titania support. All of the catalysts were prepared in a glove box in a nitrogen atmosphere to prevent decomposition of the transition metal oxide precursors. In all cases 10 grams of the P-25 titania powder were slurried in 100 cc of ethanol to which was added the transition metal oxide precursor, with the resulting mixture stirred overnight, under flowing nitrogen, to evaporate the ethanol. Each dry mixture was then taken out of the glove box and 3 cc of water added. The resulting mixture was stirred overnight in air, then the dry powder placed in a quartz boat and slowly heated in a 1/1 flowing mixture of $O_2$ in He up to 400°C. At 400°C the He flow was cut off and the powdered catalyst precursor then heated from 400 to 575°C in 100% $O_2$. Each sample of catalyst precursor was held at 575°C in the $O_2$ for two hours to calcine the precursor into a catalyst of this invention.

The transition metal oxide precursors were obtained from Alfa, Inc. and were $Nb(C_2H_5O)_5$, and $Ta(C_2H_5O)_5$. The amounts of niobia and tantala precursors added to each slurry of 10 g P-25 in 100 cc of ethanol were 2.5 and 1.84 grams, respectively. The resulting catalysts contained 10 wt.% niobia on titania and 10 wt.% tantala on titania. The niobia and tantala contents of the catalysts are expressed as niobium pentoxide and tantalum pentoxide.

In this experiment a 2 gram sample of each catalyst was charged to the reactor which was then heated up to a temperature of 200°C in flowing helium. After the reactor achieved a temperature of 200°C, a 1/1 molar mixture of $H_2S/CO$ feed at a flow rate of about 4 cc/min. was introduced to the reactor. The reactor was then held at isothermal conditions in order to establish steady-state conditions with respect to feed conversion and product selectivity. The temperaure was then raised at 50°C intervals and held at each temperature for one hour to measure activity and selectivity. The final temperature reached was 400°C. The reactor was then cooled to 300°C to recheck the activity compared to the activity during the heating step.

The results are shown in the Table and clearly demonstrate that the catalysts were effective for the conversion of the $H_2S/CO$ feed.

H$_2$S AND CO CONVERSION AS A FUNCTION OF TEMPERATURE[a]

| Catalyst | 10% Nb$_2$O$_5$/TiO$_2$ | 10% Ta$_2$O$_5$/TiO$_2$ |
|---|---|---|
| Surface Area[b] | 45m$^2$/g | 54m$^2$/g |
| Catalyst Weight | 2.0g | 2.0g |
| CO Conversion (%) | | |
| 300°C | -- | 9% |
| 300°C | 28% | 19% |
| 350°C | 40% | 33% |
| 400°C | 40% | 33% |
| H$_2$S Conversion (%) | | |
| 300°C | -- | 2.5 |
| 300°C | 10 | 8 |
| 350°C | 20 | 16 |
| 400°C | 20 | 18 |

Notes:  a)  Products included CH$_3$SH and CH$_4$
        b)  BET
        c)  After cooling from 400°C

CLAIMS:

1. A catalyst composition comprising an oxide of a metal selected from (a) niobium, (b) mixtures of niobium and tantalum, and (c) tantalum, the metal oxide being supported on a titania support wherein at least a portion of the supported metal oxide is in a non-crystalline form.

2. A composition according to claim 1 wherein at least 25 wt % of said supported metal oxide is in a non-crystalline form.

3. A composition according to either of claims 1 and 2 wherein the amount of said supported metal oxide present on said catalyst ranges from about 0.5 to 25 wt % of the catalyst composition.

4. A composition according to claim 3 wherein the amount of said supported metal oxide ranges from about 1 to 15 wt % of the catalyst composition.

5. A composition according to claim 4 wherein said supported oxide is a mixture of oxides of niobium and tantalum.

6. A composition according to claim 4 wherein the supported oxide is niobium oxide.

7. A composition according to claim 4 wherein the supported oxide is tantalum oxide.

European Patent Office

**EUROPEAN SEARCH REPORT**

Application number

EP 85 30 4586

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | BULLETIN OF THE CHEMICAL SOCIETY OF JAPAN, vol. 56, no. 7, July 1983, pages 2159-2160, Japan Publication Trading Co. Ltd., Tokyo, JP; S. OKAZAKI et al.: "Nb2O5 supported on TiO2. Catalytic activity for reduction of NO with NH3" * Page 2159, figure 2; page 2160, left-hand column * | 1-4,6 | B 01 J 23/20 B 01 D 53/36 C 07 C 148/00 C 07 C 1/02 |
| | --- | | |
| A | US-A-4 269 737 (D.C. GRENOBLE et al.) * Column 3, lines 5-59; column 5, lines 28-32; column 10, lines 30-56 * | 1-6 | |
| | --- | | |
| A | CHEMICAL ABSTRACTS, vol. 88, no. 16, 17th April 1978, page 292, no. 109945e, Columbus, Ohio, US; & JP - A - 77 145 383 (ONO HISASHI) 03-12-1977 * Abstract * | 1 | TECHNICAL FIELDS SEARCHED (Int. Cl.4) B 01 J B 01 D |
| | ----- | | |

The present search report has been drawn up for all claims

| Place of search | Date of completion of the search | Examiner |
|---|---|---|
| THE HAGUE | 03-09-1985 | KERRES P.M.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82